# EUROPÄISCHE PATENTSCHRIFT

(11) **EP 0 790 232 B1**
(45) Veröffentlichungstag und Bekanntmachung des Hinweises auf die Patenterteilung: **21.06.2000**
(21) Anmeldenummer: 97101599.5
(22) Anmeldetag: 03.02.1997
(51) Int. Cl.: C07C 209/72

(54) **Verfahren zur Herstellung eines Gemisches aus Cyclohexylamin und Dicyclohexylamin**
Process for the preparation of a mixture of cyclohexyl amine and dicyclohexyl amine
Procédé de préparation d'un mélange de cyclohexylamine et dicyclohexylamine

(30) Priorität: 15.02.1996 DE 19605585
(43) Veröffentlichungstag der Anmeldung: 20.08.1997
(73) Patentinhaber: BAYER AG, 51368 Leverkusen (DE)
(72) Erfinder: Darsow, Gerhard, Dr., 47804 Krefeld (DE); Niemeier, Wilfried, Dr., 47803 Krefeld (DE); Petruck, Gerd-Michael, Dr., 40699 Erkrath (DE)

(56) Entgegenhaltungen:
- EP-A- 0 501 265
- EP-A- 0 503 347
- DE-C- 765 846
- FR-A- 2 300 617

## Beschreibung

Die Erfindung betrifft ein kontinuierliches Verfahren zur Herstellung eines Gemisches von gegebenenfalls substituiertem Cyclohexylamin und gegebenenfalls substituiertem Dicyclohexylamin in variablen Mengen durch katalytische Hydrierung von gegebenenfalls substituiertem Anilin mit Wasserstoff bei erhöhter Temperatur und erhöhtem Druck unter Einsatz von durch Reduktion von Formkörpern aus verpreßten Pulvern von Kobalt-, Mangan- und Erdalkalimetall(hydr)oxiden sowie (Hydr)oxiden von Elementen der V. oder VI. oder beiden Nebengruppen des Periodensystem der Elemente (Mendelejeff) erhaltenen Festbettkatalysatoren. Gegebenenfalls substituierte Cyclohexylamine und Dicyclohexylamine finden Verwendung zur Herstellung von Alterungsschutzmitteln für Kautschuke und Kunststoffe, als Korrosionsschutzmittel in wäßriger Lösung, sowie als Vorprodukte für Textilhilfsmittel und Pflanzenschutzmittel.

Es ist bekannt, Cyclohexylamin durch Druckhydrierung von Anilin herzustellen. Für diese Hydrierung werden hauptsächlich kostspielige Edelmetall-Katalysatoren eingesetzt, beispielsweise gemäß US 3.636.108 ein mit Alkali moderierter Ru-Katalysator, wobei noch zusätzlich NH₃ und gegebenenfalls ein Lösungsmittel eingesetzt werden. Ein weiteres Verfahren zur Druckhydrierung von Anilin zu Cyclohexylamin ist in DE-B 1 106 319 beschrieben, wobei ebenfalls ein Ru-Katalysator verwendet wird. In diesem Verfahren wird mitentstehendes Dicyclohexylamin dem Einsatzstoff wieder zugesetzt. Das Verfahren erreicht jedoch wegen der gleichzeitigen Entstehung von Cyclohexan nur eine mäßige Ausbeute. Nach EP-B 53 818 sind Pd-Trägerkatalysatoren günstiger als Ru-Katalysatoren; die dort beschriebenen Katalysatoren enthalten Zusätze, die entweder einer Gruppe von basischen Verbindungen der Alkalimetalle, Erdalkalimetalle und Seltenerdmetalle entstammen oder einer Gruppe, welche die Metalle Fe, Ni, Co, Mn, Zn, Cd und Ag umfaßt. Diese Katalysatoren erlauben die Reduktion von substituierten Anilinen zu den zugehörigen Cyclohexylaminen; die zugehörigen Dicyclohexylamine fehlen jedoch völlig. Dies gilt gleichermaßen für Co-Katalysatoren, die einen basischen Zusatz enthalten (GB 969 542) sowie für Raney-Co (JP 68/03 180).

Bei den beschriebenen Druckhydrierungsverfahren von Anilin entsteht das Dicyclohexylamin neben dem Cyclohexylamin lediglich als Nebenprodukt oder überhaupt nicht. Um Dicyclohexylamin in größeren Mengen zu erhalten, wird es nach separaten Verfahren hergestellt. So kann es beispielsweise durch Druckhydrierung von Diphenylamin unter Verwendung eines Ru/Al₂O₃-Katalysators gewonnen werden (obige DE-B 1 106 319). Weiterhin entsteht Dicyclohexylamin bei der Umsetzung von Cyclohexanon mit Cyclohexylamin in Gegenwart von Pd auf Kohle unter einem Wasserstoffdruck von 4 bar (FR 1 530 477).

In einem umständlichen Verfahren kann Dicyclohexylamin aus dem Hydrierprodukt des Anilins an einem Ni-Katalysator durch fraktioniertes Auskondensieren gewonnen werden. Aus dem zurückbleibenden Gemisch wird ein Teil des mitentstandenen Ammoniaks entfernt und der Rest wieder in die Reaktion zurückgeführt (DE-C 805 518).

Ein gemeinsames Problem aller dieser Verfahren zur Kernhydrierung aromatischer Amine besteht in der z.T. beträchtlichen Bildung von Cyclohexan als nicht weiter zu verwendendem Nebenprodukt. Es bestand daher nach wie vor der Wunsch, ein neues, auch im technischen Maßstab brauchbares Verfahren zu entwickeln, nach welchem sowohl Cyclohexylamin als auch Dicyclohexylamin in einem gewünschten Mengenverhältnis hergestellt werden können, bei welchem der Verlust durch die unerwünschte Bildung von Cyclohexan zurückgedrängt wird und bei welchem weiterhin die Lebensdauer des verwendeten Katalysators verbessert ist.

Aus EP-A 501 265 ist ein Verfahren bekannt, gegebenenfalls substituiertes Cyclohexylamin und gegebenenfalls substituiertes Dicyclohexylamin durch katalytische Hydrierung von gegebenenfalls substituiertem Anilin herzustellen, wobei ein Katalysator eingesetzt wird, der Ru, Pd oder ein Gemisch beider Metalle enthält, die auf einem Träger aus Niobsäure oder Tantalsäure oder auf einem Gemisch beider aufgebracht sind. Aus EP-A 503 347 ist ein weiteres Verfahren bekannt, gegebenenfalls substituiertes Cyclohexylamin und gegebenenfalls substituiertes Dicyclohexylamin durch Hydrierung eines entsprechend substituierten Anilins herzustellen, wobei ein Katalysator eingesetzt wird, bei dem ein α- oder γ-Al₂O₃ als Träger zunächst mit mindestens einer Verbindung von Seltenen Erdmetallen und mit mindestens einer Verbindung des Mangans behandelt wird und danach mit mindestens einer Pd-Verbindung behandelt wird. Die Herstellung der Katalysatoren in diesen Verfahren von EP-A 501 265 und EP-A 503 347 ist technisch aufwendig und ihr Einsatz teuer, da die Wiedergewinnung der Edelmetallanteile aus den komplexen Substanzgemischen nach Erschöpfung des Katalysators erhebliche Probleme schafft, die anfangs nicht erkannt worden waren. Außerdem ist in diesem Verfahren das Mengenverhältnis der herstellbaren cyclischen Amine zu sehr in die Richtung höherer Anteile an Dicyclohexylamin verschoben. Schließlich ist die Lebensdauer der in den zuletzt genannten Verfahren eingesetzten Katalysatoren mit.3 000 bis 4 000 h zu gering, so daß sie die in sie gesetzten Erwartungen nicht erfüllen konnten.

Überraschenderweise wurde jetzt gefunden, daß die obengenannten Anforderungen durch den Einsatz preiswerter oxidischer Festbettkatalysatoren, die frei sind von inaktivem Trägermaterial und daher einfach aufgearbeitet und entsorgt werden können, erfüllt werden.

Die Erfindung betrifft somit ein Verfahren zur Herstellung eines Gemisches aus Cyclohexylamin und Dicyclohexylamin der Formeln 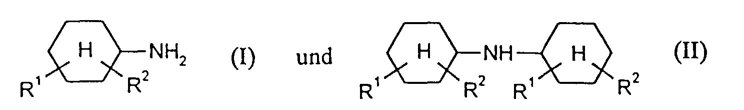 durch katalytische Hydrierung von Anilin der Formel 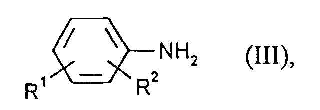 wobei in den Formeln
- R¹ und R²: unabhängig voneinander Wasserstoff, C₁-C₄-Alkyl oder C₁-C₄-Alkoxy bedeuten,
bei einer Reaktionstemperatur von 140 bis 240°C und einem H₂-Druck von 10 bis 400 bar, das dadurch gekennzeichnet ist, daß als Katalysatoren dienende, trägerfreie Festkörper eingesetzt werden, die durch Reduktion von Formkörpern aus verpreßten Metall(hydr)oxid pulvern folgender Zusammensetzung erhältlich sind : Co-Anteile (jeweils gerechnet als Metall) 40 bis 65 Gew.-%, Mn-Anteile 10 bis 20 Gew.-%, Erdalkalimetall-Anteile 0,2 bis 5 Gew.-% und Anteile an Elementen der V. und/oder VI. Nebengruppe des Periodensystems insgesamt 0,5 bis 5 Gew.-%. Der Rest zu 100 Gew.-% ist Sauerstoff. Cu kann in einer Menge von 0-3 Gew.-% vorliegen.

Von den Erdalkalielementen eignen sich vor allem Magnesium, Calcium, Strontium und Barium, vorzugsweise Strontium und Barium. Von den Elementen der V. Nebengruppe eignen sich vorzugsweise Vanadium, Niob und Tantal, von den Elementen der VI. Nebengruppe vorzugsweise Chrom, Molybdän und Wolfram. Die als Promotoren wirkenden Elemente der V. und VI. Nebengruppe können entweder einzeln oder als Mischung mehrerer dieser Elemente zum Einsatz kommen.

Zur Herstellung der Katalysatoren werden Pulver von Oxiden oder Hydroxiden der genannten Elemente eingesetzt. In bevorzugter Weise werden. Oxidpulver der genannten Elemente eingesetzt. Solche Pulver werden in den Mengen mechanisch miteinander vermischt, daß sie die oben angegebenen Gewichtsverhältnisse erfüllen. Der Rest zu 100 Gew.-% ist stets der Anteil des Sauerstoffs, und alle Gewichtsprozente sind auf das Gesamtgewicht des oxidischen, trägerfreien Formkörpers bezogen. Das Gemisch der Pulver wird sodann auf Tablettier- oder Pelletier-Maschinen unter hohem Druck verpreßt, wobei zur Verbesserung des Haftvermögens der Pulver auch Graphit, Klebstoffe oder beide in Mengen von 0,5-1 Gew.-%, bezogen auf das Gesamtgewicht der zu verpressenden Pulver, zum Einsatz kommen können. Beispiele für die Form solcher Preßlinge sind Tabletten, Kugeln oder zylindrische Granulate mit Abmessungen von 1-10 mm, bevorzugt 3-7 mm. Tablettierte Formkörper können weiterhin zur Vergrößerung der äußeren Oberfläche mit einer axialen Durchbohrung versehen sein. Solche verpreßten Formkörper haben makroskopisch betrachtet eine glatte Oberfläche. Die verpreßten Formkörper haben eine hohe Druckfestigkeit auf die Formkörperoberfläche. So haben Tabletten oder zylindrische Granulate auf die ebenen Preßoberflächen eine Druckfestigkeit von 200-800 N/cm², bevorzugt 250-600 N/cm² bei Benutzung eines flächigen Druckstempels, und Tabletten, Kugeln oder zylindrische Granulate haben eine Druckfestigkeit auf die gewölbten Preßoberflächen von 50-200 N (gemessen als Kraft), bevorzugt 80-140 N bei Benutzung eines messerförmigen Druckgebers. Die innere Oberfläche der eingesetzten verpreßten Formkörper beträgt 30-200 m²/g, bevorzugt 80-160 m²/g. Die Druckfestigkeit der trägerfreien Formkörper kann nach DIN 50 106 bestimmt werden. Die Bestimmung der inneren Oberfläche erfolgt nach F.M. Nelsen und F.T. Eggertsen, Analyt. Chem. 30 (1958), S. 1387-1390 bzw. nach S. J. Gregg und K.S.W. Sing, Adsorption Surface Area and Porosity, Academic Press, London 1982, Kap. 2 + 6.

Mit Hilfe des Einsatzes der beschriebenen Katalysatoren entsteht im erfindungsgemäßen Verfahren ein Gemisch von gegebenenfalls substituiertem Cyclohexylamin und gegebenenfalls substituiertem Dicyclohexylamin, wobei überraschend in Abhängigkeit von der Hydriertemperatur das Mengenverhältnis beider Amine so verändert werden kann, daß mit steigender Temperatur mehr gegebenenfalls substituiertes Dicyclohexylamin und weniger gegebenenfalls substituiertes Cyclohexylamin gebildet wird und mit sinkender Temperatur der umgekehrte Effekt erreicht wird.

Der Temperaturbereich für das erfindungsgemäße Verfahren beträgt 140-240°C, bevorzugt 160-230°C. Es wird bei einem H₂-Druck von 10-400 bar, bevorzugt 20-350 bar, besonders bevorzugt 100-300 bar, gearbeitet.

Das erfindungsgemäße Verfahren kann mit den im Festbett angeordneten Katalysatoren kontinuierlich in der Gas- oder in der Rieselphase durchgeführt werden, wobei während des Verfahrensablaufs mindestens die 10fache molare Menge Wasserstoff pro Mol Ausgangsmaterial den Reaktor passiert. Bevorzugt wird in der Rieselphase gearbeitet. Die Hydrierreaktoren können einzelne Hochdruckrohre aus Stahl oder einer Stahllegierung sein, die mit den Formkörpern ganz oder teilweise gefüllt werden, wobei bei größeren Rohrquerschnitten auch die Anwendung der trägerfreien Formkörper auf Horden, wie Drahtkörben oder ähnlichen Einbauten, nützlich sein kann. Weiterhin kann man auch Hochdruck-Rohrbündel innerhalb eines gemeinsamen Mantels anwenden, wobei die Einzelrohre wiederum ganz oder teilweise mit dem Katalysatorformkörpern gefüllt werden.

Die verpreßten, trägerlosen Katalysatoren werden durch Wasserstoff reduziert und damit aktiviert. Dies ist grundsätzlich simultan zur Hydrierung des eingesetzten Ausgangsmaterials möglich, wobei jedoch eine längere Einlaufphase erforderlich ist, bevor die Katalysatoren ihre volle Aktivität erreichen und damit die höchstmögliche Raum-Zeit-Ausbeute eintritt. Es ist daher vorteilhaft, den Katalysator vor der Beschickung mit Ausgangsmaterial zu reduzieren. Diese aktivierende Reduktion mit Wasserstoff wird im Temperaturbereich von 160-240°C und im Druckbereich von 10-400 bar durchgeführt. Hierbei wird zunächst durch ein Inertgas, wie Stickstoff, Argon, Methan oder Ethan der zunächst vorhandene Luftsauerstoff vollständig entfernt, bevor dem Inertgas ein Anteil von 10-15 Vol.-% Wasserstoff zugesetzt wird. Das Inertgas ist aus Gründen der guten Verfügbarkeit bevorzugt Stickstoff. Innerhalb eines festgesetzten Zeitraums, beispielsweise von 24 h, wird sodann der Anteil an Inertgas ständig vermindert und schließlich das Inertgas ganz entfernt, so daß mit reinem Wasserstoff aktiviert und reduziert wird. Die Reduktion ist beendet, wenn der Katalysator keinen Wasserstoff mehr verbraucht und infolge dessen kein Reaktionswasser mehr bildet.

Bei der Verfahrensweise in Rieselphase beträgt die Katalysatorbelastung 0,1-3 kg, bevorzugt 0,15-1,5 kg, gegebenenfalls substituiertes Anilin pro Liter Katalysator und Stunde. Das eingesetzte, gegebenenfalls substituierte Anilin kann mit einem geeigneten reaktionsinerten Lösungsmittel, beispielsweise mit Cyclohexan oder Cyclohexanol in einer Menge von 10-100, bevorzugt 10-40 Gew.-%, bezogen auf das Gewicht des gegebenenfalls substituierten Anilins, verdünnt werden. Durch den Zusatz von Cyclohexanol in der angegebenen Menge, das auch ganz oder teilweise durch Cyclohexanon oder Phenol ersetzt werden kann, ist es weiterhin möglich, das im Verfahren freigesetzte Ammoniak durch Aminierung abzufangen und weiteres gegebenenfalls substituiertes Cyclohexylamin/Dicyclohexylamin zu bilden. Bei der kontinuierlichen Fahrweise in der Rieselphase kann es günstig sein, das eingesetzte, gegebenenfalls substituierte Anilin, nicht vollständig zu hydrieren, sondern einen Umsetzungsgrad von 80-97 % anzusteuern.

Die erfindungsgemäß eingesetzten Katalysatoren zeigen sehr hohe Standzeiten; bisher sind 12 000 bis 15 000 h beobachtet worden, bei denen Versuche ohne erkennbares Nachlassen der Aktivität abgebrochen wurden. Diese Standzeiten sind um ein Vielfaches höher, als sie in den obengenannten EP-A 501 265 und EP-A 503 347 beschrieben sind.

Als Einsatzmaterial für das erfindungsgemäße Verfahren kommen gegebenenfalls substituierte Aniline der obigen Formel (III) in Frage. C₁-C₄-Alkyl und C₁-C₄-Alkoxy als gegebenenfalls vorhandene Substituenten sind beispielsweise Methyl, Ethyl, Propyl, Isopropyl, Butyl, Isobutyl, Methoxy, Ethoxy, Propoxy, Isopropoxy, Butoxy, Isobutoxy. Von diesen sind Methyl, Ethyl, Methoxy und Ethoxy als gegebenenfalls vorhandene Substituenten bevorzugt. In besonders bevorzugter Weise haben die Substituenten die Bedeutung Methyl oder Methoxy. In weiterhin bevorzugter Weise ist R² Wasserstoff, während R¹ den genannten Bedeutungsumfang annehmen kann. In ganz besonders bevorzugter Weise wird nichtsubstituiertes Anilin zu nichtsubstituiertem Cyclohexylamin und nicht substituiertem Dicyclohexylamin hydriert.

Die nach der Hydrierung erhaltenen Reaktionsgemische enthalten kein Cyclohexan, sofern dieses nicht als Lösungsmittel hinzugefügt wurde, so daß besonders hohe Gehalte an gegebenenfalls substituiertem Cyclohexylamin und gegebenenfalls substituiertem Dicyclohexylamin erzielt werden können. Die Hydriergemische können durch einfache Destillation aufgearbeitet werden. Für eine solche Aufarbeitung kann es vorteilhaft sein, das gegebenenfalls substituierte Anilin nicht vollständig umzusetzen. Das nicht vollständig umgesetzte Anilin kann wieder in die Reaktion zurückgeführt werden. Auch der nichtverbrauchte Anteil des in 10-80fachem molaren Überschuß zugesetzten Wasserstoffs kann in die Reaktion zurückgeführt werden, wobei in vorteilhafter Weise der größte Teil dieses nicht umgesetzten Wasserstoffs in einem Hochdruckabscheider zurückgewonnen wird, so daß die Kompressionsarbeit für den Wasserstoff nicht noch einmal geleistet zu werden braucht.

Das erfindungsgemäß hergestellte gegebenenfalls substituierte Cyclohexylamin und das gegebenenfalls substituierte Dicyclohexylamin werden nach erfolgter destillativer Trennung in einer Reinheit von mindestens 99,9 Gew.-% erhalten. In dieser Reinheit sind die genannten Verbindungen in der Regel für alle weiterverarbeitenden Prozesse einsetzbar.

Die Variabilität des erfindungsgemäßen Verfahrens zeigt sich in einer starken Zunahme des Anteils an gegebenenfalls substituiertem Dicyclohexylamin gegenüber dem gegebenenfalls substituiertem Cyclohexylamin mit steigender Temperatur unter sonst gleichen Bedingungen. So erhält man beispielsweise eine Steigerung des Anteils an gegebenenfalls substituiertem Dicyclohexylamin im Temperaturbereich von etwa 180-230°C auf das 2-10fache des Anteils im Temperaturbereich von 170-175°C.

### Beispiele

### Beispiel 1

Ein senkrecht stehendes, wärmeisoliertes Hochdruckrohr aus nichtrostendem, säurefestem Stahl von 90 mm Innendurchmesser und 1,8 m Länge, das vorher mit Stickstoff sauerstofffrei gespült worden war, wurde mit 11,4 l eines durch Tablettierung von Pulvern von Co-, Mn-, Ba-, und Mo-Oxiden hergestellten Hydrierungskatalysators gefüllt. Der Co-Gehalt der Tabletten lag bei 53 Gew.-%, der Mn-Gehalt bei 14 Gew.-%, der Ba-Gehalt bei 1,1 Gew.-% und der Mo-Gehalt bei 1,2 Gew.-% (Rest zu 100 %: Sauerstoff). Die Tabletten hatten bei einer Zylinderhöhe von 5 mm und einem Durchmesser von 5 mm eine Druckfestigkeit von 420 N/cm² auf die Querschnittsfläche und von 125 N, gemessen als Kraft, auf die Zylindermantelfläche sowie eine innere Oberfläche von 168 m²/g.

Die Tabletten wurden zunächst 6 Stunden im Stickstoffstrom getrocknet (Temperatur: max. 200°C, Menge: 5 Nm³N₂/h). Die Aktivierung erfolgte dann unter einem Stickstoffdruck von 200 bar bei einer Temperatur zwischen 180 und 240°C, wobei dem Stickstoff allmählich Wasserstoff zugemischt wurde, dessen Zumischungsanteil anfangs 10 bis 15 Vol.-% betrug. Innerhalb von 24 Stunden wurde der Stickstoffanteil der Gasmischung mehr und mehr vermindert, bis schließlich reiner Wasserstoff den Reaktor durchströmte. Die Aktivierung war beendet, sobald sich kein Reaktionswasser im nachgeschalteten Abscheider mehr ansammelte. Nach der Aktivierung des Katalysators wurde der Wasserstoffdruck im Reaktorsystem auf 300 bar erhöht. Anschließend wurden stündlich in der Rieselphase 2.280 g Anilin gemeinsam mit 70 Nm³ Wasserstoff unter einem Druck von 300 bar durch das Hochdruckrohr gepumpt, wobei das zu hydrierende Anilin vor Eintritt in das erste Hochdruckrohr in einem vorgeschalteten elektrisch beheizten Wärmeaustauscher auf eine Temperatur von 160°C erhitzt wurde. Das das Reaktionsrohr verlassende Reaktionsprodukt wurde in einem zweiten Wärmeaustauscher (Wasserkühler) unter 300 bar Wasserstoffdruck auf eine Temperatur <60°C abgekühlt und in einem Gasabscheider vom überschüssigen Wasserstoff, der wieder in das Hydriersystem zurückgeführt werden konnte, getrennt. Nach weiterer Abkühlung auf eine Temperatur <30°C und Entspannung auf Normaldruck wurde das Reaktionsprodukt gaschromatographisch untersucht. Bei stationären Reaktionsbedingungen ergab sich folgende Produktzusammensetzung in Abhängigkeit von den Reaktionstemperaturen (Angabe in Flächen-(F)%, CHA = Cyclohexylamin, DCHA = Dicyclohexylamin, der Rest zu 100 % sind Anilin und Nebenprodukte; einzelne Laufzeiten bei den jeweiligen Temperaturen):

| Laufzeit (h) | Temperatur (°C) | CHA (F-%) | DCHA (F-%) |
|---|---|---|---|
| 500 | 175 | 86,8 | 3,7 |
| 452 | 180 | 89,3 | 7,5 |
| 404 | 185 | 86,5 | 12,7 |
| 116 | 190 | 77,3 | 21,8 |
| 212 | 200 | 71,6 | 27,0 |
| 236 | 210 | 62,9 | 34,1 |
| 308 | 220 | 54,8 | 38,5 |
| 380 | 230 | 46,9 | 40,3 |

### Beispiel 2

Ein Hochdruckrohr wie in Beispiel 1, das vorher mit Stickstoff sauerstofffrei gespült worden war, wurde mit 11,4 l eines durch Tablettierung von Pulvern von Co-, Mn-, Cu-, Ba- und V-Oxiden hergestellten Hydrierungskatalysators gefüllt. Der Co-Gehalt der Tabletten lag bei 52 Gew.-%, der Mn-Gehalt bei 16 Gew.-%, der Cu-Gehalt bei 0,18 Gew.-%, der Ba-Gehalt bei 0,91 Gew.-% und der V-Gehalt bei 1,1 Gew.-% (Rest zu 100 Gew.-% Sauerstoff). Die Tabletten hatten bei einer Zylinderhöhe von 7 mm und einem Durchmesser von 7 mm eine Druckfestigkeit von 420 N/cm² auf die Querschnittsfläche und von 160 N auf die Zylindermantelfläche sowie eine innere Oberfläche von 180 m²/g. Die Tabletten wurden wie in Beispiel 1 getrocknet und aktiviert. Nach der Aktivierung des Katalysators wurde der Wasserstoffdruck im Reaktorsystem auf 300 bar erhöht. Anschließend wurden stündlich in der Rieselphase 2.280 gh Anilin gemeinsam mit 70 Nm³ Wasserstoff unter einem Druck von 300 bar durch das Hochdruckrohr gepumpt, wobei das zu hydrierende Anilin vor Eintritt in das erste Hochdruckrohr in einem vorgeschalteten elektrisch beheizten Wärmeaustauscher auf eine Temperatur von 160°C erhitzt wurde. Das das Reaktionsrohr verlassende Reaktionsprodukt wurde in einem zweiten Wärmeaustauscher (Wasserkühler) unter 300 bar Wasserstoffdruck auf eine Temperatur <60°C abgekühlt und in einem Gasabscheider vom überschüssigen Wasserstoff, der wieder in das Hydriersystem zurückgeführt wurde, getrennt. Nach weiterer Abkühlung auf eine Temperatur <30°C und Entspannung auf Normaldruck wurde das Reaktionsprodukt gaschromatographisch untersucht. Bei stationären Reaktionsbedingungen ergab sich folgende Produktzusammensetzung in Abhängigkeit von den Reaktionstemperaturen (Angaben wie in Beispiel 1; einzelne Laufzeiten bei den jeweiligen Temperaturen):

| Laufzeit (h) | Temperatur (°C) | CHA (F-%) | DCHA (F-%) |
|---|---|---|---|
| 525 | 190 | 92,8 | 5,00 |
| 94 | 200 | 89,2 | 10,20 |
| 142 | 210 | 80,5 | 17,95 |
| 166 | 220 | 78,8 | 18,00 |
| 223 | 230 | 69,1 | 24,30 |
| 240 | 240 | 55,8 | 28,40 |

### Beispiel 3

Ein senkrecht stehendes, wärmeisoliertes Hochdruckrohr aus nichtrostendem, säurefestem Stahl von 45 mm Innendurchmesser und 1 m Länge, das vorher mit Stickstoff sauerstofffrei gespült worden war, wurde mit 1,4 l eines durch Tablettierung von Pulvern von Co-, Mn-, Cu-, Sr- und W-Oxiden hergestellten Formkörpers gefüllt. Der Co-Gehalt der Tabletten lag bei 53 Gew.-%, der Mn-Gehalt bei 14 Gew.-%, der Cu-Gehalt bei 0,2 Gew.-%, der Sr-Gehalt bei 0,9 Gew.-% und der W-Gehalt bei 1,1 Gew.-% (Rest zu 100 Gew.-% Sauerstoff). Die Tabletten hatten bei einer Zylinderhöhe von 6 mm und einem Durchmesser von 6 mm eine Druckfestigkeit von 533 N/cm² auf die Querschnittsfläche und von 162 N auf die Zylindermantelfläche sowie eine innere Oberfläche von 142 m²/g.

Die Tabletten wurden wie in Beispiel 1 getrocknet und aktiviert. Nach der Aktivierung des Hydrierkatalysators wurde der Wasserstoffdruck im Reaktorsystem auf 300 bar erhöht. Anschließend wurden stündlich 560 g Anilin gemeinsam mit 10 Nm³ Wasserstoff unter einem Druck von 300 bar von oben nach unten absteigend durch das Hochdruckrohr gepumpt, wobei das Anilin vor Eintritt in den Reaktor in einem vorgeschalteten elektrisch beheizten Wärmeaustauscher auf eine Temperatur von 160°C erhitzt wurde. Das das Reaktionsrohr verlassende Reaktionsprodukt wurde in einem zweiten Wärmeaustauscher (Wasserkühler) unter 300 bar Wasserstoffdruck auf eine Temperatur <60°C abgekühlt und in einem Gasabscheider vom überschüssigen Wasserstoff, der wieder in das Hydriersystem zurückgeführt werden konnte, getrennt. Nach weiterer Abkühlung auf eine Temperatur <30°C und Entspannung auf Normaldruck wurde das Reaktionsprodukt gaschromatographisch untersucht. Bei stationären Reaktionsbedingungen ergab sich folgende Produktzusammensetzung in Abhängigkeit von den Reaktionstemperaturen (Angaben wie in Beispiel 1; kumulierte Laufzeiten).

| Laufzeit (h) | Temperatur (°C) | CHA (F-%) | DCHA (F-%) |
|---|---|---|---|
| 64 | 180 | 84,2 | 8,5 |
| 118 | 190 | 78,6 | 19,7 |
| 542 | 200 | 74,1 | 29,4 |
| 812 | 210 | 68,6 | 31,1 |
| 1112 | 220 | 64,1 | 35,2 |
| 1422 | 230 | 54,1 | 43,8 |

### Beispiel 4

Ein Hochdruckrohr wie in Beispiel 1 wurde mit 11,4 1 eines durch Tablettierung von Pulvern von Co-, Mn-, Cu-, Ba-, und Mo-Oxiden hergestellten Hydrierungskatalysators gefüllt. Der Co-Gehalt der Tabletten lag bei 53 Gew.-%, der Mn-Gehalt bei 14 Gew.-%, der Cu-Gehalt bei 0,2 Gew.-%, der Ba-Gehalt bei 0,9 Gew.-% und der Mo-Gehalt bei 1,1 Gew.-%. Die Tabletten hatten bei einer Zylinderhöhe von 6 mm und einem Durchmesser von 6 mm eine Druckfestigkeit von 425 N/cm² auf die Querschnittsfläche und von 160 N auf die Zylindermantelfläche sowie eine innere Oberfläche von 180 m²/g. Nach der Trocknung und Aktivierung des oxidischen Hydrierkatalysators wie in Beispiel 1 wurde der Wasserstoffdruck auf 300 bar erhöht. Anschließend wurden stündlich in der Rieselphase 2.280 g Anilin gemeinsam mit 70 Nm³ Wasserstoff unter einem Druck von 300 bar durch das Hochdruckrohr gepumpt, wobei das zu hydrierende Anilin vor Eintritt in das Hochdruckrohr in einem vorgeschalteten, elektrisch beheizten Wärmeaustauscher auf eine Temperatur von 200°C erhitzt wurde. Das das Reaktionsrohr verlassende Reaktionsprodukt wurde in einem zweiten Wärmeaustauscher (Wasserkühler) unter 300 bar Wasserstoffdruck auf eine Temperatur <60°C abgekühlt und in einem Gasabscheider vom überschüssigen Wasserstoff, der wieder in das Hydriersystem zurückgeführt wurde, getrennt. Nach weiterer Abkühlung auf eine Temperatur <30°C und Entspannung auf Normaldruck wurde das Reaktionsprodukt gaschromatographisch untersucht. Bei stationären Reaktionsbedingungen ergab sich folgende Produktzusammensetzung (Angaben wie in Beispiel 1; kumulierte Laufzeiten).

| Laufzeit (h) | Temperatur (°C) | CHA (F-%) | DCHA (F-%) |
|---|---|---|---|
| 182 | 199 | 71,6 | 27,0 |
| 2.978 | 198 | 32,9 | 28,4 |
| 5.412 | 205 | 66,8 | 32,8 |
| 8.216 | 208 | 62,6 | 35,8 |
| 14.458 | 210 | 62,8 | 36,2 |

## Patentansprüche

1. Verfahren zur Herstellung eines Gemisches aus Cyclohexylamin und Dicyclohexylamin der Formeln 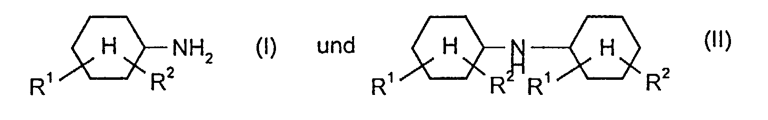 durch katalytische Hydrierung von Anilin der Formel 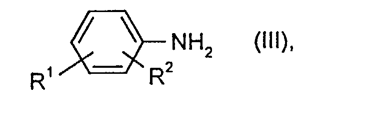 wobei in den Formeln
R¹ und R² unabhängig voneinander Wasserstoff, C₁-C₄-Alkyl oder C₁-C₄-Alkoxy bedeuten,
bei einer Reaktionstemperatur von 140 bis 240°C und einem H₂-Druck von 10 bis 400 bar, dadurch gekennzeichnet, daß als Katalysator dienende, trägerfreie Formkörper eingesetzt werden, die durch Reduktion von Formkörpern aus verpreßten Metall(hydr)oxidpulvern erhältlich sind, die 40 bis 65 Gew.-% Kobalt, 10 bis 20 Gew.-% Mangan, 0,2 bis 5 Gew.-% Erdalkalimetall, 0 bis 3 Gew.-% Kupfer und 0,2 bis 5 Gew.-% Elemente der V. und/oder VI. Nebengruppe des Periodensystems (jeweils gerechnet als Metalle) enthalten, wobei sich die Prozentangaben auf die Gesamtmenge Metall(hydr)oxid-Pulvergemisch beziehen und der Rest zu 100 Gew.-% Sauerstoff ist.

2. Verfahren nach Anspruch 1, dadurch gekennzeichnet, daß die Katalysatoren eine Druckfestigkeit von 200-800 N/cm², bevorzugt 250-600 N/cm², auf ebene Preßoberflächen, eine Druckfestigkeit von 50-200 N, gemessen als Kraft, bevorzugt 80-140 N, auf gewölbte Preßoberflächen und eine innere Oberfläche von 30-200 m²/g, bevorzugt 80-160 m²/g, aufweisen.

3. Verfahren nach Anspruch 1, dadurch gekennzeichnet, daß kontinuierlich in der Rieselphase an fest angeordneten Katalysatoren gearbeitet wird und eine Katalysatorbelastung von 0,1-3 kg, bevorzugt von 0,15-1,5 kg, Anilin pro Liter Katalysator und Stunde eingestellt wird.

4. Verfahren nach Anspruch 1, dadurch gekennzeichnet, daß bei einem H₂-Druck von 20-350 bar, bevorzugt 100-300 bar, gearbeitet wird.

5. Verfahren nach Anspruch 1, dadurch gekennzeichnet, daß bei einer Temperatur von 160-230°C gearbeitet wird.

6. Verfahren nach Anspruch 1, dadurch gekennzeichnet, daß die Katalysatoren vor ihrem Einsatz durch Behandlung mit Wasserstoff bei 160-240°C und 10-400 bar reduziert werden, wobei der Wasserstoff zu Beginn der Reduktion als H₂/Inertgas-Gemisch eingesetzt wird und der Inertgasanteil im Verlauf der Reduktion vollständig entfernt wird.

7. Verfahren nach Anspruch 1, dadurch gekennzeichnet, daß das gegebenenfalls substituierte Anilin mit 10-100 Gew.-%, bevorzugt 10-40 Gew.-%, eines reaktionsinerten Lösungsmittels, bezogen auf das gegebenenfalls substituierte Anilin, verdünnt wird.

## Claims

1. Process for preparing a mixture of cyclohexylamine and dicyclohexylamine of the formulae; 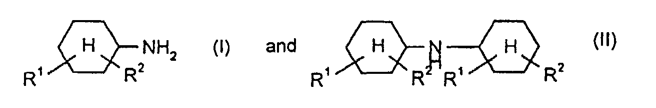 by catalytic hydrogenation of aniline of the formula 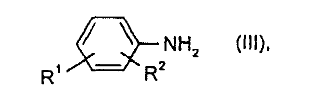 where, in the formulae,
R¹ and R² are, independently of one another, hydrogen, C₁-C₄-alkyl or C₁-C₄-alkoxy,
at a reaction temperature of from 140 to 240°C and an H₂ pressure of from 10 to 400 bar, characterized in that the catalyst used comprises support-free shaped bodies which are obtainable by reduction of shaped bodies comprising pressed metal (hydr)oxide powders containing from 40 to 65 % by weight of cobalt, from 10 to 20 % by weight of manganese, from 0.2 to 5 % by weight of alkaline earth metal, from 0 to 3% by weight of copper and from 0.2 to 5 % by weight of elements of transition groups V and/or VI of the Periodic Table (in each case calculated as metals), where the percentages are based on the total amount of metal (hydr)oxide powder mixture and the remainder to 100 % by weight is oxygen.

2. Process according to Claim 1, characterized in that the catalysts have a compressive strength of 200-800 N/cm², preferably 250-600 N/cm², on flat surfaces, a compressive strength of 50-200 N, measured as force, preferably 80-140 N, on curved surfaces and an internal surface area of 30-200 m²/g, preferably 80-160 m²/g.

3. Process according to Claim 1, characterized in that the reaction is carried out continuously in a trickling phase over fixed-bed catalysts and the weight hourly space velocity used is 0.1-3 kg, preferably 0.15-1.5 kg, of aniline per litre of catalyst and hour.

4. Process according to Claim 1, characterized in that the reaction is carried out at an H₂ pressure of 20-350 bar, preferably 100-300 bar.

5. Process according to Claim 1, characterized in that the reaction is carried out at a temperature of 160-230°C.

6. Process according to Claim 1, characterized in that the catalysts are reduced before use by treatment with hydrogen at 160-240°C and 10-400 bar, with the hydrogen being used as an H₂/inert gas mixture at the beginning of the reduction and the inert gas component being completely removed during the course of the reduction.

7. Process according to Claim 1, characterized in that the unsubstituted or substituted aniline is diluted with 10-100 % by weight, preferably 10-40 % by weight, of a reaction-inert solvent, based on the unsubstituted or substituted aniline.

## Revendications

1. Procédé pour la préparation d'un mélange de cyclohexylamine et de dicyclohexylamine répondant aux formules 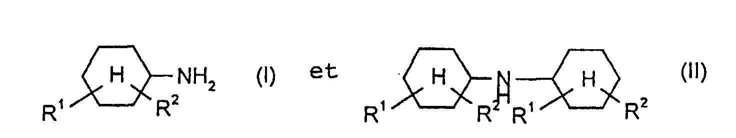 par hydrogénation catalytique d'aniline répondant à la formule 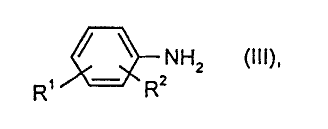 dans lequel, dans les formules,
R¹ et R² représentent, indépendamment l'un de l'autre, un atome d'hydrogène, un groupe alkyle en C₁-C₄ ou un groupe alcoxy en C₁-C₄,
à une température réactionnelle de 140 à 240°C et sous une pression de H₂ de 10 à 400 bar, caractérisé en ce qu'on met en oeuvre des corps moulés exempts de supports faisant office de catalyseurs que l'on obtient par réduction de corps moulés constitués par des poudres d'(hydr)oxydes métalliques comprimées qui contiennent du cobalt à concurrence de 40 à 65% en poids, du manganèse à concurrence de 10 à 20% en poids, un métal alcalino-terreux à concurrence de 0,2 à 5% en poids, du cuivre à concurrence de 0 à 3% en poids et des éléments des sous-groupes V et/ou VI du système périodique (chaque fois calculés comme métaux) à concurrence de 0,2 à 5% en poids, les indications en pour cent se rapportant à la quantité totale du mélange pulvérulent d'(hydr)oxydes métalliques, le reste pour obtenir 100% en poids étant de l'oxygène.

2. Procédé selon la revendication 1, caractérisé en ce que les catalyseurs présentent une résistance à la pression de 200 à 800 N/cm², de préférence de 250 à 600 N/cm² sur des surfaces de compression planes, une résistance à la pression de 50 à 200 N, mesurée comme force, de préférence de 80 à 140 N sur des surfaces de compression bombées et une surface interne de 30 à 200 m²/g, de préférence de 80 à 160 m²/g.

3. Procédé selon la revendication 1, caractérisé en ce qu'on travaille en continu dans la phase de ruissellement sur des catalyseurs disposés en lit fixe et on règle une charge du catalyseur de 0,1 à 3 kg, de préférence de 0,15 à 1,5 kg d'aniline par litre de catalyseur et par heure.

4. Procédé selon la revendication 1, caractérisé en ce qu'on travaille sous une pression de H₂ de 20 à 350 bar, de préférence de 100 à 300 bar.

5. Procédé selon la revendication 1, caractérisé en ce qu'on travaille à une température de 160 à 230°C.

6. Procédé selon la revendication 1, caractérisé en ce qu'on réduit les catalyseurs avant leur mise en oeuvre par traitement avec de l'hydrogène à une température de 160 à 240°C et sous une pression de 10 à 400 bar, l'hydrogène étant mis en oeuvre au début de la réduction sous la forme d'un mélange de H₂/gaz inerte et la fraction de gaz inerte étant complètement éliminée au cours de la réduction.

7. Procédé selon la revendication 1, caractérisé en ce qu'on dilue l'aniline le cas échéant substituée avec un solvant inerte vis-à-vis de la réaction à concurrence de 10 à 100% en poids, de préférence à concurrence de 10 à 40% en poids rapportés à l'aniline le cas échéant substituée.
